# EUROPEAN PATENT APPLICATION

(11) **EP 0 541 493 A1**
(43) Date of publication of application: **12.05.1993**
(21) Application number: 92830558.0
(22) Date of filing: 05.10.1992
(51) Int. Cl.: A61K 6/027, C04B 40/00, C04B 28/14

(54) **Process for the production of a plaster model in dentistry, and products for carrying out this process**

(30) Priority: 07.10.1991 IT FI910240
(71) Applicant: Cozzi, Gualtiero, I-50137 Firenze (IT)
(72) Inventor: Cozzi, Gualtiero, I-50137 Firenze (IT)
(74) Representative: Mannucci, Gianfranco, Dott.-Ing.

(57) **Abstract**

For the production of plaster models in dentistry, there are added to the plaster-based material at least one further component having basic characteristics and at least one further component having acid characteristics, these components being soluble in water, together with a pH indicator in order to obtain indications on the course of the process from the time of the mixing with water, the variation in the pH of the mixture being correlated with this course.

## Description

It is known in dentistry that, for the preparation and finishing of prostheses, an impression of the dentition is taken - partial or complete, depending on the type of prosthesis to be made - and subsequently plaster mixed with water in suitable proportions is cast on the impression for the purpose of reproducing the dentition of the patient with a plaster model. For the material to be used in the taking of the impression, the characteristics required are elasticity, dimensional stability and toughness. The plaster cannot be a plaster selected at will. In fact, the plaster must be of great fineness, must expand very little during setting and must be capable of being mixed with a small quantity of water in order to achieve a hardened state which is compact and abrasion-resistant. Furthermore, the following characteristics of the plaster must be known: the mixing time, in order that the components which are present as setting correctors can be brought completely into solution; the time within which the casting on the impression can be carried out; and the time to be allowed for completion of setting, in order to be able to detach the model from the impression without incurring the risk of fracture.

These operations leading to the preparation of the model would give good results if the various times could be monitored by means of a chronometer or at least a watch; but this is inconvenient in everyday dental laboratory practice and disadvantages frequently arise. For example, if the mixing stage is performed for too short a time, the dissolution of the correcting components may be incomplete; in this case the setting is non-uniform and the expansion during setting may not be negligible. If the casting is delayed too long, there is an increase in the viscosity at the start of setting; in this case perfect distribution of the plaster over the surface of the model may become difficult if not impossible. If the stage of setting and hardening is not allowed sufficient time to reach completion, fracture of the model may occur at the time of its detachment from the impression. These disadvantages, resulting from minor inaccuracies or oversights, may be at least in part eliminated by the invention, which can be integrated with the invention covered by Application FI/91/A/13 of January 18, 1991.

Substantially, the process according to the invention for the production of plaster models in dentistry provides that there are added to the plaster-based material a further component having basic characteristics and a further component having acid characteristics - both being soluble in water - together with a pH indicator; this provides indications on the course of the process from the time of the mixing with water, the variation in the pH of the mixture being correlated with this course.

Another subject of the invention is a product for the production of plaster models in dentistry which comprises, mixed with the plaster-based material, a further component having basic characteristics and a further component having acid characteristics, both being soluble in water, together with a pH indicator capable - in use - of supplying indications as to the course of the process from the time of the mixing with water, the variation in the pH of the mixture being correlated with this course.

Said further components and the proportions thereof in the plaster may be selected as a function of their rate of dissolution, in order to achieve variations from basic to neutral pH, from acid to neutral pH, from basic to acid pH and also from acid to basic pH, respectively.

One or more chromatic pH indicators may be used, the coloration whereof - a function of the pH - displays variations in color on passing from the stage of mixing and application to the stage of setting and hardening, and at the end of this latter stage.

The invention relates to the process and the product for the production of plaster models in dentistry by the carrying out of the abovementioned process.

As is known, in the preparation of the plaster for impressions, corrective components are introduced by the manufacturer to regulate the time and the expansion during setting. According to the invention, there are added to the plaster both a further component having an alkaline character (inorganic or organic) and low solubility, such as to impart to the plaster/water mixture a pH greater than 7, and a substance having an acid character (inorganic or organic) and low solubility, such as to impart to the plaster/water mixture a pH less than 7. These substances become active at the time of the mixing with water, while they remain unaffected by the storage of the plaster until the operation of use.

In a possible embodiment, if the two sparingly soluble substances, one having an alkaline character and the other an acid character, are present in the plaster in equal quantities - expressed in terms of equivalents - that substance which has the higher solubility exerts the principal initial action and imparts its features to the mixture. Thus, for example, if the acid component is the more soluble, the plaster/water mixture is of a pH less than 7 and remains so until the two components have mutually neutralized each other. The mixture then returns to neutrality.

In another possible embodiment, the acid component has an action which initially predominates over the alkaline component and the latter is present in a greater quantity than the other, still expressed in terms of equivalents. A mixture is then obtained which has a pH less than 7 while all the component having an acid character is present. As soon as the latter has disappeared as a result of the neutralization produced by the component having an alkaline character, the pH of the mixture rises to above 7. In this case the plaster/water mixture is initially acid but slowly, over the course of time, becomes neutral and then alkaline.

Again, in another possible embodiment, if the basic component has the predominant action, the pH is greater than 7 initially and less than 7 ultimately.

In any event, the variation in pH can be clearly displayed if a coloring substance is present which is commonly known under the name of "pH indicator".

According to a possible form of implementation, the product may be prepared in a manner such that, at a predetermined stage, the pH indicator is present in a particular color other than that of the subsequent stage.

The sparingly soluble substance having an acid character may, for example, be sodium fluorosilicate (m.w. 188.05); this salt reacts as follows in aqueous solution:

Si F₆⁼ 4 + H₂O → Si (OH)₄ + 4H⁺ + 6F⁻

The substance having an alkaline character may, for example, be magnesium oxide (m.w. 40.32). This is very sparingly soluble and, in water, occurs in dissociated form as Mg⁺⁺20H⁻. Taking into account the molecular weights and the associated equivalents, it is easily possible to calculate that one part by weight of sodium fluorosilicate is completely neutralized by 0.43 part by weight of magnesium oxide. With ratios of less than 1:0.43, for example 1:1 or 1:2, the initially acid plaster/water mixture slowly becomes alkaline.

By way of example, a preparation such as the following may be provided:

| | |
|---|---|
| Sodium fluorosilicate | 0.2% |
| Phenolphtalein | 0.01% |
| Magnesium oxide | 0.4% |
| Type III plaster | q.s.p. 100 g |

This preparation, mixed with water in the ratio of 100 g per 28 g of water, provides a white mixture which remains so for 4 minutes and 30 seconds. After this time, the composition takes on a pink color, and at 5 minutes setting has begun. The pink color tends to fade until hardening is complete, which occurs after about 20 minutes. Decoloration takes place at the same time. With this mixture, therefore, three stages may be identified:
I - a first, white stage, during which the plaster is mixed and may be cast on the impression carrier;
II - a second, pink stage, corresponding to the process of setting and hardening, during which the plaster must be in position on the impression and must be kept there undisturbed;
III - a third stage of decoloration, which indicates that hardening has taken place, it being possible for the model to be separated from the impression when this stage is reached.

As possible examples of compounds having an acid character which may be used, the following may be indicated: the fluorosilicates and analogous compounds such as fluorotitanates and fluorozirconates; sparingly soluble inorganic acids such as tungstic acid; sparingly soluble organic acids; slowly hydrolyzable organic esters; and others.

As compounds of basic character which may be used, the following may be indicated: oxides and hydroxides of metals such as magnesium, zinc and others; sparingly soluble organic bases.

As pH indicators it is possible to use all those whose range of action lies between pH 4 and pH 10.

It is understood that what has been set out above is merely an example, given solely by way of a practical demonstration of the invention, the present invention being capable of varying in respect of forms and arrangements without thereby departing from the scope of the concept which underlies said invention.

## Claims

1. A process for the production of plaster models in dentistry, wherein there are added to the plaster-based material a further component having basic characteristics and a further component having acid characteristics, both being soluble in water, together with a pH indicator, in order to obtain indications on the course of the process from the time of the mixing with water, the variation in the pH of the mixture being correlated with this course.

2. The process as claimed in claim 1, wherein said further components and the proportions thereof in the plaster are selected as a function of their rate of dissolution, in order to achieve variations from basic to neutral pH.

3. The process as claimed in claim 1, wherein said further components and the proportions thereof in the plaster are selected as a function of their rate of dissolution, in order to achieve variations from acid to neutral pH.

4. The process as claimed in claim 1, wherein said further components and the proportions thereof in the plaster are selected as a function of their rate of dissolution, in order to achieve variations from basic to acid pH.

5. The process as claimed in claim 1, wherein said further components and the proportions thereof in the plaster are selected as a function of their rate of dissolution, in order to achieve variations from acid to basic pH.

6. The process as claimed in one or more of the preceding claims, wherein, in order to reveal the pH, one or more chromatic indicators are used whose color, a function of the pH, serves to indicate the stage of mixing and application, the stage of hardening, and the completion of the latter.

7. The process as claimed in one or more of the preceding claims, wherein a component having aci d characteristics is selected from: fluorosilicates and analogous compounds, such as fluorotitanates and fluorozirconates; sparingly soluble inorganic acids such as tungstic acid; sparingly soluble organic acids; slowly hydrolyzable organic esters.

8. The process as claimed in one or more of claims 1 to 7, wherein a component having basic characteristics is selected from: oxides and hydroxides of metals, such as magnesium, zinc and others; sparingly soluble organic bases.

9. The process as claimed in the preceding claims, wherein a pH indicator is selected from among those whose range of action is between pH 4 and pH 10.

10. A product for the production of plaster models in dentistry, for the carrying out of the process as claimed in one or more of claims 1 to **9**.

11. A product for the production of plaster models in dentistry, comprising, mixed with the plaster-based material, a further component having basic characteristics and a further component having acid characteristics, both being soluble in water, together with a pH indicator capable - in use - of supplying indications as to the course of the process from the time of the mixing with water, the variation in the pH of the mixture being correlated with this course.

12. The product as claimed in claim **11,** wherein said further components and the proportions thereof in the plaster are selected as a function of their rate of dissolution, in order to achieve variations from basic to neutral pH.

13. The product as claimed in claim **11,** wherein said further components and the proportions thereof in the plaster are selected as a function of their rate of dissolution, in order to achieve variations from acid to neutral pH.

14. The product as claimed in claim **11,** wherein said further components and the proportions thereof in the plaster are selected as a function of their rate of dissolution, in order to achieve variations from basic to acid pH.

15. The product as claimed in claim **11,**wherein said further components and the proportions thereof in the plaster are selected as a function of their rate of dissolution, in order to achieve variations from acid to basic pH.

16. The product as claimed in one or more of claim **11** and the subsequent claims, comprising a chromatic pH indicator whose coloration tends to disappear on completion of the course of the process.

17. The product as claimed in one or more of claim **11** and the subsequent claims, wherein a component having acid characteristics is selected from: fluorosilicates and analogous compounds, such as fluorotitanates and fluorozirconates; sparingly soluble inorganic acids such as tungstic acid; sparingly soluble organic acids; slowly hydrolyzable organic esters.

18. The product as claimed in one or more of claim **11** and the subsequent claims, wherein a component having basic characteristics is selected from: oxides and hydroxides of metals, such as magnesium, zinc and others; sparingly soluble organic bases.

19. The product as claimed in claim **11** and the subsequent claims, wherein a pH indicator is selected from among those whose range of action is between pH 4 and pH 10.
